# EUROPÄISCHE PATENTSCHRIFT

(11) **EP 1 955 729 B1**
(45) Veröffentlichungstag und Bekanntmachung des Hinweises auf die Patenterteilung: **15.01.2014**
(21) Anmeldenummer: 07023315.0
(22) Anmeldetag: 01.12.2007
(51) Int. Cl.: A61N 1/05, H01R 13/52, H01R 107/00

(54) **Elektrodenleitung und Anschlussstück für einen implantierbaren Herzstimulator**
Electrode wire and connector piece for an implantable heart stimulator
Ligne d'électrodes et élément de raccord pour un stimulateur cardiaque implantable

(30) Priorität: 07.02.2007 DE 102007006089
(43) Veröffentlichungstag der Anmeldung: 13.08.2008
(73) Patentinhaber: Biotronik CRM Patent AG, 6341 Baar (CH)
(72) Erfinder: Bartels, Klaus, Dr., 10115 Berlin (DE); Steglich, Carsten, 12587 Berlin (DE); Thomas, Günther, 14557 Wilhelmshorst (DE)
(74) Vertreter: Lindner-Vogt, Karin L.

(56) Entgegenhaltungen:
- US-A- 5 304 219
- US-A1- 2005 221 671
- US-A1- 2007 027 517
- US-B1- 6 439 933

## Beschreibung

Die Erfindung betrifft eine Elektrodenleitung zum Anschluss an einen implantierbaren Herzstimulator wie beispielsweise einen Herzschrittmacher oder einen Cardioverter/Defibrillator. Insbesondere betrifft die Erfindung ein Anschlussstück für eine solche Elektrodenleitung.

Implantierbare Herzstimulatoren der genannten Art sind in verschiedenen Ausführungen grundsätzlich bekannt. Derartige Herzstimulatoren weisen üblicherweise ein dichtes Gehäuse auf, welches eine Stromversorgung und weitere elektronische und elektrische Bauteile umfasst, um Stimulationsimpulse oder Defibrillationsschocks zu erzeugen und über eine Elektrodenleitung an eine oder mehrere Kammern eines Herzens abzugeben oder um elektronische Potentiale im Herzen zu erfassen und zu verarbeiten. Zu diesem Zweck sind solche implantierbaren Herzstimulatoren üblicherweise mit Elektrodenleitungen verbunden, die mit einem Anschlussstück am proximalen Ende der Elektrodenleitung in eine Buchse in einem sogenannten Header des jeweiligen Herzstimulators eingesteckt sind. Sowohl das Anschlussstück als auch die Buchse im Header des Herzstimulators weisen miteinander korrespondierende elektrische Kontakte auf, um eine elektrisch leitende Verbindung zwischen dem Herzstimulator und der Elektrodenleitung herzustellen.

Dementsprechend weist das Anschlussstück am proximalen Ende der Elektrodenleitung elektrische Kontakte auf, die mit Anschlussleitungen verbunden sind, welche wenigstens den Teil einer elektrischen Verbindung zu Stimulations- und/oder Abfühlelektroden im Bereich des distalen Endes einer solchen Elektrodenleitung herstellen. Solche Elektrodenleitungen sind üblicherweise flexibel (also biegeweich) und in mannigfacher Ausführung bekannt.

Die vorliegende Erfindung gilt dem Anschlussstück am proximalen Ende der Elektrodenleitung und dessen Verbindung zu übrigen Elektrodenleitungen.

Das hier betroffene Anschlussstück ist dabei von der Art, die mehrere, ringförmige Kontakte gleichen Außendurchmessers aufweist und der zukünftigen Norm IS-4 entspricht. Eine Variante eines derartigen Anschlussstücks ist beispielsweise in US 2005/0221671 beschrieben.

Das Dokument US-A-2005/221671 offenbart den nächstliegenden Stand der Technik.

Aufgabe der Erfindung ist es, ein Anschlussstück und eine Elektrodenleitung mit einem solchen Anschlussstück zu schaffen, die unabhängig von der Art und Zahl der Kontakte der Elektrodenleitung sind, mit vertretbaren Aufwand herzustellen sind und eine hohe Zuverlässigkeit besitzen.

Erfindungsgemäß wird diese Aufgabe durch ein Anschlussstück sowie durch eine Elektrodenleitung mit einem Anschlussstück gelöst, das mehrere ringförmige, elektrisch leitende Kontakte gleichen Außendurchmesser mit in Längsrichtung dazwischen angeordneten Isolationsabschnitten desselben Außendurchmesser aufweist, von denen die elektrisch leitenden Kontakte mit jeweils einer elektrisch leitenden Anschlussleitung elektrisch leitend verbunden sind. Das erfindungsgemäße Anschlussstück ist von mehreren isolierenden Zwischenstücken und von von diesen gehaltenen elektrischen Kontakten mit daran befestigten Anschlussleitungen gebildet, in Längsrichtung des Anschlussstücks zusammengesteckt und nach dem Zusammenstecken mit isolierenden Kunststoff umspritzt. Dabei ragen die Anschlussleitungen entweder gar nicht oder höchstens um ein Maß über ein distales des Anschlussstücks hinaus, welches geringer ist, als die übrige Länge des Anschlussstücks

Die Zwischenstücke sind vorzugsweise als Vorspritzlinge mittels Spritzguss hergestellt.

Im Übrigen ist das erfindungsgemäße Anschlussstück so ausgebildet, dass es als eigenständige Einheit mit einer übrigen Elektrodenleitung zu verbinden ist.

Dies erlaubt es, dass vollständige Anschlussstück vorzufertigen und zu prüfen, bevor es mit der übrigen Elektrodenleitung verbunden wird. Damit kann ein fehlerhaftes Anschlussstück schon identifiziert werden, bevor es mit der übrigen Elektrodenleitung verbunden wird. Im Falle eines Fehlers am Anschlussstück produzierter Ausschuss betrifft somit nicht die gesamte Elektrodenleitung.

Für das Umspritzen erforderliche Angüsse und zwingend notwendige Trennebenen von Spritzgussformen sind so gelegt, dass eine äußere Oberfläche des isolierenden Kunststoffes im Bereich zwischen den einzelnen elektrischen Kontakten und proximal des am weitesten proximal angeordneten Kontaktes keine Werkzeugmarken aufweisen. Aus praktischen Erwägungen wie beispielsweise aus Anforderungen, die sich aus der Biokompatibilität ergeben, kann das Umspritzen auch aus mehreren Schritten mit differierenden Materialien geschehen, so dass sich eine heterogene Außenstruktur ergibt, die die Zwischenstücke zumindest teilweise umschließt.

Alternativ hierzu können die Zwischenstücke so ausgebildet und in Längsrichtung zusammengesteckt sein, dass sie zusammen mit den elektrischen Kontakten die Außenkontur des Anschlussstücks bilden und ein von den Zwischenstücken umschlossener Innenraum des Anschlussstücks mit isolierendem Kunststoff ausgespritzt ist.

In beiden Fällen dienen die Zwischenstücke sowohl der axialen als auch radialen Lagesicherung der elektrisch leitenden Kontakte.

Um eine rationelle Fertigung zu gewährleisten und die Zuverlässigkeit des Anschlussstücks zu erhöhen, ist es vorgesehen, dass wenigstens zwei der isolierenden Zwischenstücke einander identisch sind. Dies bringt den weiteren Vorteil mit sich, dass das Anschlussstück ohne weiteres mit einer, zwei oder drei ringförmigen Kontakten aufgebaut werden kann, ohne dass hierzu andere Bauteile nötig wären.

Vorzugsweise besitzen die identischen Zwischenstücke jeweils einen passgenauen Sitz für einen jeweiligen ringförmigen Kontakt. Dies bedeutet, dass das jeweilige Zwischenstück wenigstens in einem Längsabschnitt eine äußere Abmessung besitzt, die dem Innendurchmesser der ringförmigen Kontakte entspricht.

Im Zusammenhang mit der zuletzt genannten Ausführungsvariante ist es vorteilhaft, wenn die einander identischen Zwischenstücke in einem jeweiligen proximalen Längsabschnitt einen Längsanschlag besitzen, der ein Aufschieben eines jeweiligen ringförmigen Kontaktes in proximale Richtung begrenzt. Dabei hat der Längsabschnitt vorzugsweise einen Längsabstand zu einem proximalen Ende eines jeweiligen Zwischenstücks, der dem Längsabstand zwischen den ringförmigen Kontakten entspricht und damit die Isolationslänge zwischen den ringförmigen Kontakten definiert.

In einem distalen Längsabschnitt weisen die einander identischen Zwischenstücke einen geringeren Außendurchmesser auf als in ihrem proximalen Längsabschnitt. Dabei ist der Außendurchmesser des distalen Längsabschnitts so bemessen, dass ein jeweiliger distaler Längsabschnitt genau in eine zentrale Öffnung im proximalen Längsabschnitt eines identischen Zwischenstücks passt, so dass die Zwischenstücke auf diese Weise ineinander zu stecken sind. Durch Ineinanderstecken der aufeinander folgenden Zwischenstücke entsteht somit ein zusammenhängender Träger für die ringförmigen Kontakte der keines weiteren Trägerelementes bedarf und die ringförmigen Kontakte sowohl radial als auch axial fixiert. Somit erlauben es die Zwischenstücke auf einfache Art und Weise einen Träger für die Kontakte herzustellen, der hinsichtlich seiner Länge und der Anzahl der Kontakte flexibel ist.

Hierbei bildet jeweils ein Abschnitt des jeweiligen proximalen Längsabschnitts eines jeweiligen Zwischenstückes mit seinem relativ größeren Außendurchmesser den Sitz für einen jeweiligen ringförmigen Kontakt.

Vorzugsweise weisen die Zwischenstücke auf ihrer Außenseite wenigstens eine in Längsrichtung verlaufende Vertiefung auf, die der Aufnahme einer jeweiligen Anschlussleitung eines ringförmigen Kontaktes dient.

Neben den Zwischenstücken besitzt das Anschlussstück vorzugsweise ein distales Abschlussstück, welches einen proximalen Endabschnitt und einen distalen Endabschnitt besitzt. Beide Endabschnitte weisen vorzugsweise annähernd den gleichen Außendurchmesser auf. Der proximale Endabschnitt des distalen Abschlussstücks entspricht vorzugsweise einem jeweiligen proximalen Längsabschnitt der Zwischenstücke zwischen dem proximalen Ende des jeweiligen Zwischenstücks und dem Längsanschlag, d. h. die proximalen Endabschnitte aller Zwischenstücke und des distalen Abschlussstücks gleichen sich.

Auch das distale Abschlussstück weist in seinem proximalen Endabschnitt vorzugsweise wenigstens eine in Längsrichtung verlaufende Vertiefung zur Aufnahme einer Anschlussleitung eines ringförmigen Kontaktes auf. Dabei ist das distale Abschlussstück im Übrigen nicht zur Aufnahme eines weiteren ringförmigen Kontaktes ausgebildet, sondern vielmehr dazu, mit der übrigen Elektrodenleitung verbunden zu werden.

Die zusammengesteckten Zwischenstücke und das Abschlussstück sind vorzugsweise so zueinander ausgerichtet, dass die jeweiligen Vertiefungen auf der Außenseite der Zwischenstücke und des Abschlussstücks miteinander fluchten und es erlauben, dass eine jeweilige Anschlussleitung gestreckt und parallel zur Längsachse des Anschlussstücks in den Vertiefungen geführt ist. Dazu sind in einem Übergangsbereich der Zwischenstücke zwischen deren proximalem Längsabschnitt mit größerem Außendurchmesser und deren distalem Längsabschnitt mit geringerem Außendurchmesser Durchbrüche in Verlängerung einer jeweiligen Vertiefung vorgesehen, die es erlauben, dass eine jeweilige Anschlussleitung diesen Übergangsbereich eines jeweiligen Zwischenstücks durchdringt.

Vorzugsweise sind in jedem Zwischenstück und im distalen Abschlussstück zwei oder drei der Vertiefungen gleichmäßig über den Umfang verteilt vorgesehen. Dabei sind die Vertiefungen in den proximalen Längsabschnitten größeren Durchmessers der Zwischenstücke gegenüber den Vertiefungen in den distalen Längsabschnitten in Umfangsrichtung versetzt.

Ein weiteres vorteilhaftes Merkmal eines bevorzugten Anschlussstücks ist ein zentrales Lumen, das sich zentral durch alle identischen Zwischenstücke sowie auch durch das Abschlussstück hindurch erstreckt und ein proximales Ende einer beispielsweise wendelförmigen elektrischen Leitung zentral aufnehmen kann.

Darüber hinaus ist am proximalen Ende des Anschlussstücks ein Segment vorgesehen, welches in die Öffnung am proximalen Ende desjenigen der identischen Zwischenstücke passgenau eingesetzt ist, welches sich am proximalen Ende des Anschlussstücks befindet. Über das Segment können verschiedenartige Elektrodenleitungskonfigurationen mit feststehender bzw. drehbarer Elektrodenleitung an das fertig gestellte Anschlussstück angebracht werden, ohne dass das Anschlussstück verändert werden muss.

Das Anschlussstück der zuvor beschriebenen Art ist vorzugsweise Bestandteil einer Elektrodenleitung, die in ihrem distalen Ende wenigstens eine Elektrode und darüber hinaus wenigstens eine elektrische Zuleitung aufweist, mit ihrem distalen Ende mit dieser Elektrode elektrisch verbunden ist und mit ihrem proximalen Ende mit einer Anschlussleistung eines ringförmigen Kontaktes des Anschlussstücks über eine Crimp-, Schweiß- oder Lötverbindung elektrisch leitend verbunden ist.

Die Crimp-, Schweiß- oder Lötverbindung befindet sich dabei vorzugsweise jeweils in einer der in Längsrichtung verlaufenden Vertiefungen des distalen Abschlussstücks des Anschlussstücks. Da das distale Abschlussstück selbst aus isolierendem Kunststoff besteht und die einzelnen elektrischen Zuleitungen und Anschlussleitungen durch die verschiedenen Vertiefungen in dem Abschlussstück voneinander elektrisch isoliert sind, ist auch nach dem Verbinden der jeweiligen Anschlussleitung mit der zugehörigen elektrischen Zuleitung keine weitere Isolierung der Verbindungsstelle erforderlich.

Wie zuvor schon angedeutet, weist die Elektrodenleitung darüber hinaus vorzugsweise eine helixartig gewendelte (wendelförmige) Leitung auf, die sich zwischen dem distalen und dem proximalen Ende der Elektrodenleitung erstreckt und sich mit einem proximalen Leitungsendabschnitt in dem Lumen des Anschlussstücks erstreckt.

Es versteht sich, dass verschiedene der vorgenannten und der in den abhängigen Ansprüchen genannten bevorzugten Merkmale des Anschlussstücks beziehungsweise der Elektrodenleitung miteinander kombiniert werden können.

Eine dem Vorstehenden entsprechende Elektrodenleitung soll nun anhand eines Ausführungsbeispiels mit Bezug auf die Figuren näher erläutert werden. Von den Figuren zeigen:
- Fig. 1:: ein als separate Einheit fertiggestelltes Anschlussstück in einer perspektivischen Außenansicht;
- Fig. 2:: eine Explosionszeichnung des Anschlussstücks aus Fig. 1 mit einer Darstellung der einzelnen Bestandteile;
- Fig. 3:: die zusammengesteckten Bestandteile des Anschlussstücks aus Fig. 1 vor dem Umspritzen.
- Fig. 4:: das Anschlussstück aus Fig. 1 nebst weiteren Bestandteilen zur Verbindung des Anschlussstücks einer übrigen Elektrodenleitung; und
- Fig. 5:: eine übrige Elektrodenleitung, die zur Verbindung mit dem Anschlussstück aus Figuren 1 bis 4 vorkonfiguriert ist.

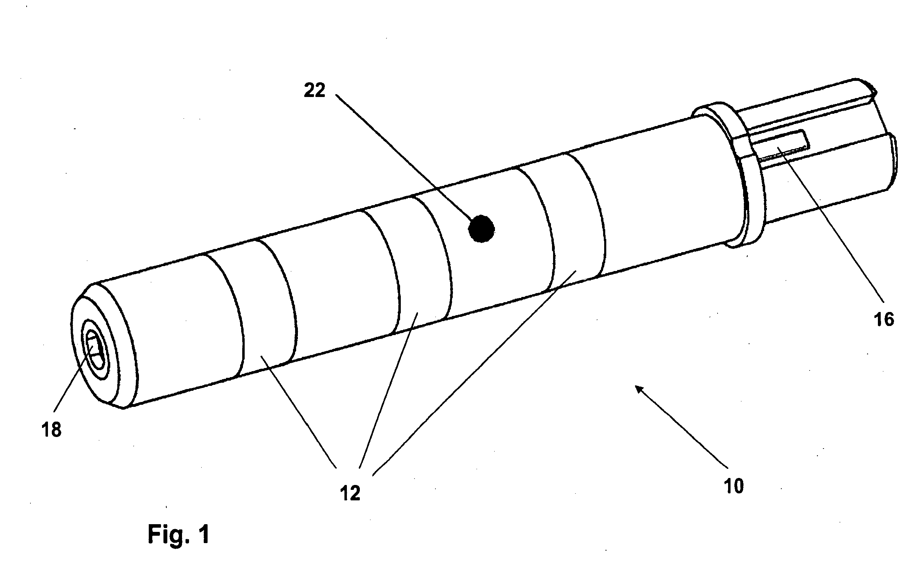

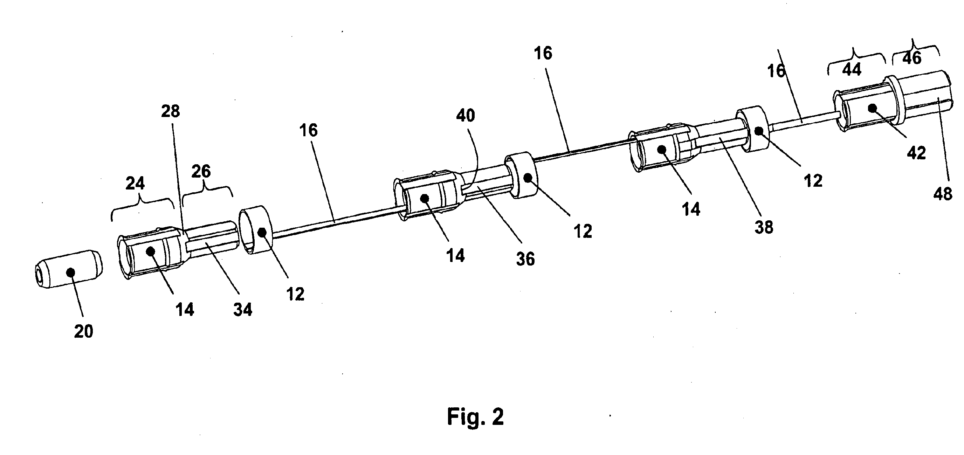

Das in Figur 1 dargestellte Anschlussstück 10 weist drei ringförmige Kontakte 12 auf, die auf einem von in Figur 1 nicht weiter erkennbaren Zwischenstücken 14 gebildeten Träger angeordnet sind. Die ringförmigen Kontakte 12 sind jeweils mit Anschlussleitungen 16 versehen, die proximal eines distalen Endes des Anschlussstücks 10 (in Figur 1 das rechte Ende des Anschlussstücks 10) enden. Am proximalen Ende des Anschlussstücks 10 befindet sich eine Schlüsselfläche 18, die Teil eines Schlüsselsegments 20 ist (siehe Figur 2). Das Anschlussstück 10 ist mit isolierendem Kunststoff umspritzt, der Isolationsflächen 22 zwischen blanken, elektrisch leitenden Außenflächen der ringförmigen Kontakte 12 bildet.

In Figur 2 sind die Bestandteile des Anschlussstücks 10 in einer Explosionszeichnung einzeln dargestellt. Insgesamt sind drei ringförmige Kontakte 12 mit an ihnen befestigten Anschlussleitungen 16 vorgesehen. Da die ringförmigen Kontakte 12, wie Figur 1 zu entnehmen ist, in Längsrichtung des Anschlussstücks 10 zueinander versetzt sind, sind die Anschlussleitungen 16 der einzelnen ringförmigen Kontakte 12 unterschiedlich lang und haben so an ihrem jeweiligen distalen Ende den gleichen Abstand zum distalen Ende des Anschlussstücks 10.

Jedes der ringförmigen Kontaktelemente 12 wird von einem jeweiligen Zwischenstück 14 getragen. Insgesamt sind drei Zwischenstücke 14 vorgesehen, die einander identisch sind. Jedes der Zwischenstücke 14 besitzt einen proximalen Längsabschnitt 24 und einen distalen Längsabschnitt 26 und einen dazwischen angeordneten Übergangsbereich 28. Ein Teil des proximalen Längsabschnitts 24 eines jeweiligen Zwischenstücks 14 dient als Sitz 30 für einen jeweiligen ringförmigen Kontakt 12. Ein Längsanschlag 22, der einen gewissen Abstand zu einem distalen Ende eines jeweiligen Zwischenstücks 12 hat, definiert den Sitz 30 für die ringförmigen Elektroden 12 in Längsrichtung des jeweiligen proximalen Längsabschnitts 24 eines jeweiligen Zwischenstücks 12.

Die jeweiligen distalen Längsabschnitte 26 eines jeweiligen Zwischenstücks 14 besitzen einen geringeren Durchmesser als die proximalen Längsabschnitte 24, der so bemessen ist, dass ein jeweiliger distaler Längsabschnitt 26 in eine zentrale Öffnung im proximalen Längsabschnitt 24 eines identischen Zwischenstücks 14 passgenau einzustecken ist. Auf diese Weise können mehrere Zwischenstücke 14 in Längsrichtung miteinander verbunden werden. Im Übergangsbereich 26 zwischen den beiden Längsabschnitten eines jeweiligen Zwischenstücks 14 verringert sich der Außendurchmesser des Zwischenstücks.

Auf der Außenseite der Längsabschnitte 24 und 26 der Zwischenstücke 14 sind in Längsrichtung verlaufende Vertiefungen 34, 36 und 38 vorgesehen. Diese Vertiefungen 34, 36 und 38 dienen der Aufnahme einer jeweiligen Anschlussleitung 16 eines ringförmigen Kontaktes 12. Dabei sind die Vertiefungen 34, 36 und 38 so angeordnet, dass eine Anschlussleitung 16 eines jeweiligen Kontaktes 12 von der Außenseite eines jeweiligen proximalen Längsabschnitts des entsprechenden Zwischenstücks 14 in einer Vertiefung (in diesem Falle jeweils die Vertiefung 38) zur Außenseite des distalen Längsabschnitts 26 des jeweiligen Zwischenstücks 14 geführt ist. Um die Anschlussleitungen 16 jeweils bis in die Nähe des distalen Endes des Anschlussstücks 10 führen zu können, sind die übrigen Vertiefungen 36 auf einem jeweiligen distalen Längsabschnitt 26 eines Zwischenstücks 14 in Verlängerung eines Durchbruchs 40 im Übergangsbereich 28 eines jeweiligen Zwischenstücks 14 angeordnet. Die Durchbrüche 40 erlauben es, dass eine jeweilige Vertiefung 38 sich in gerader Richtung in entsprechend fluchtenden Vertiefungen 36 eines oder mehrerer weiter distal angeordneter Zwischenstücke 14 fortsetzt.

Am distalen Ende des Anschlussstücks 10 ist ein Abschlussstück 42 angeordnet, das einen proximalen Endabschnitt 44 und einen distalen Endabschnitt 46 besitzt. Der proximale Endabschnitt 44 ist ähnlich gestaltet wie die proximalen Längsabschnitte 24 der Zwischenstücke 14, weist jedoch keinen Sitz für eine weitere ringförmige Elektrode auf. Der distale Endabschnitt 46 des Abschlussstücks 42 besitzt in etwa den gleichen Außendurchmesser wie der proximale Endabschnitt 44 des Abschlussstücks 42 und weist ebenfalls in Längsrichtung verlaufende Vertiefungen 48 auf. Wie Figur 1 zu entnehmen ist, enden die Anschlussleitungen 16 in einer jeweiligen dieser Vertiefungen 48, wenn das Anschlussstück 10 fertig montiert ist. Die Enden der Anschlussleitungen 16 sind dabei in den Vertiefungen 48 von außen zugänglich.

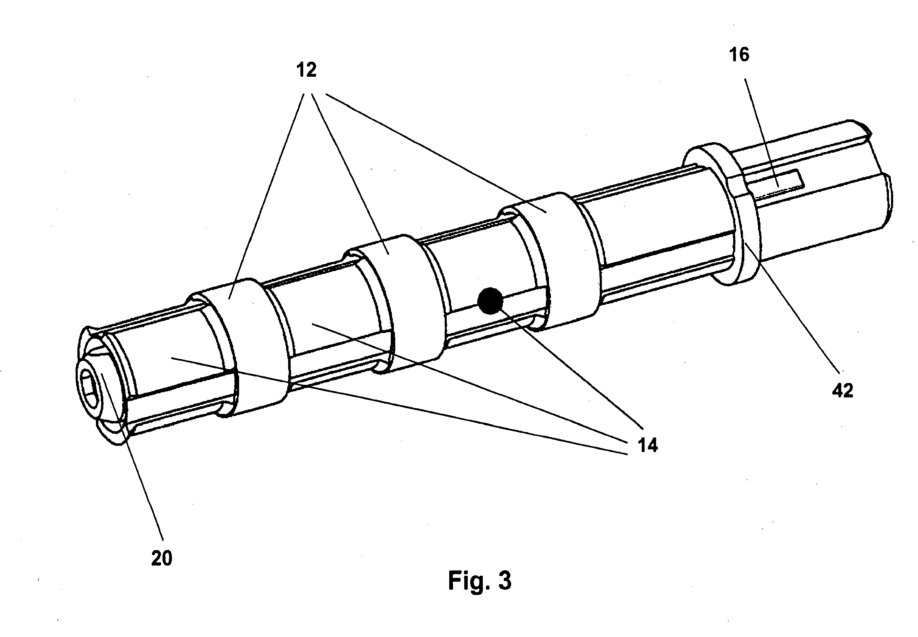

Wie bereits erwähnt, können die Zwischenstücke 14 sowie das Abschlussstück 42 in Längsrichtung ineinander gesteckt werden und bilden auf diese Weise einen Kontaktträger für die elektrischen Kontakte 12. Figur 3 zeigt ein zusammengestecktes Zwischenstück 10 vor dem Umspritzen mit isolierendem Kunststoff.

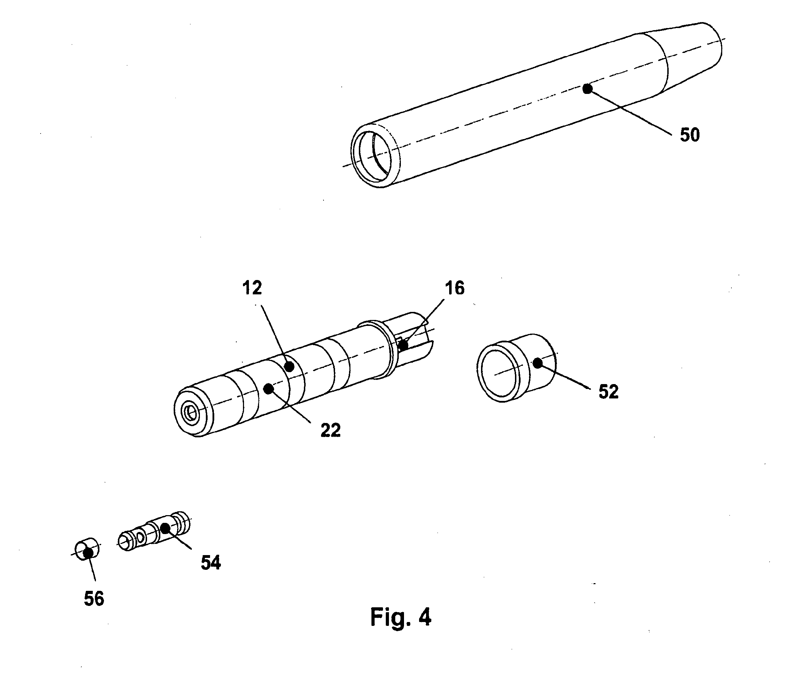

Um das fertiggestellte Anschlussstück 10 mit einer übrigen Elektrodenleitung verbinden zu können, sind weitere Teile erforderlich, die in Figur 4 dargestellt sind. Dies sind eine Silikonhülse 50, ein Abschlussring 52, ein Steckerpin 54 und ein Markierungsband 56.

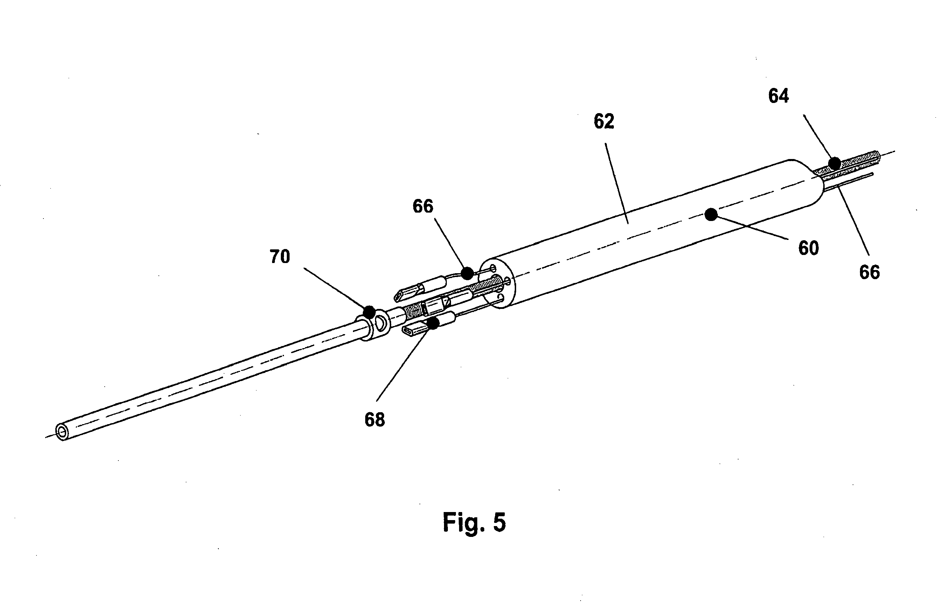

Mit Hilfe der letztgenannten Bestandteile kann das Abschlussstück 10 mit einem proximalen Ende einer insoweit vorkonfigurierten Elektrodenleitung 60 verbunden werden, wie es in Figur 5 dargestellt ist. Figur 5 zeigt nur das proximale Ende einer solchen vorkonfigurierten Elektrodenleitung 60. Wie Figur 5 zu entnehmen ist, besitzt die Elektrodenleitung 60 eine biegeweiche Hülle 62, die ein zentrales Lumen für eine zentrale, helixartig gewendelte Zuleitung 64 sowie weiter außen angeordnete Lumen für elektrische Zuleitungen 66 aufweist. Die elektrischen Zuleitungen 66 sind an ihrem jeweiligen proximalen Ende mit jeweils einer Krimphülse 68 ausgestattet, die der Verbindung der elektrischen Zuleitungen 66 mit den Anschlussleitungen 16 der ringförmigen Elektroden 12 dient. Am proximalen Ende der helixartig gewendelten Leitung 64 ist eine Hülse 70 befestigt, die sich durch ein von dem Abschlussstück 42 und den Zwischenstücken 14 eingeschlossenes, durchgängiges, zentrales Lumen des Anschlussstücks 10 erstreckt, wenn das Anschlussstück 10 an der Elektrodenleitung 60 befestigt ist. Ein proximales Ende der Hülse 70 wird dann mit dem Steckerpin 54 verbunden. Der Steckerpin 54 bildet daraufhin einen zentralen Kontakt für die helixartig gewendelte Leitung 64.

Um das fertiggestellte Anschlussstück 10 mit der Elektrodenleitung 60 zu verbinden, sind somit die folgenden Montageschritte erforderlich. Zunächst werden an den Enden der Zuleitungen 66 die Crimphülsen 68 durch Crimpen befestigt. Anschließend wird die Hülse 70 durch Laserschweißen mit dem proximalen Ende der helixartig gewendelten Leitung 64 verbunden. Das vorbereitete, in Figur 5 dargestellte proximale Ende der Elektrodenleitung 60 wird dann in das Anschlussstück 10 eingeführt und mit dem Steckerpin 54 fixiert. Die Silikonhülse 50 und der Abschlussring 52 werden dann als Verbindungselemente über die Übergangsstellen gebracht. Das Markierungsband 56 deckt eine Abschmelzkante für die Laserschweißverbindung zwischen dem Steckerpin 54 und der Hülse 70 ab.

Diese Beschreibung eines bevorzugten Ausführungsbeispiels macht deutlich, dass das Abschlussstück 10 bereits vor seiner Verbindung mit einer Elektrodenleitung 60 weitestgehend fertiggestellt und geprüft werden kann, so dass sichergestellt ist, dass das Abschlussstück 10 fehlerfrei ist, wenn es mit der Elektrodenleitung 60 verbunden wird. Dies senkt die Ausschlussquote und erhöht die Qualität der fertigen Elektrodenleitung.

## Patentansprüche

1. Anschlussstück für eine Elektrodenleitung zum Anschluss an einen implantierbaren Herzstimulator und/oder implantierbaren Defibrillator, wobei das Anschlussstück mehrere ringförmige, elektrisch leitende Kontakte (12) gleichen Außendurchmessers mit in Längsrichtung dazwischen angeordneten Isolationsabschnitten desselben Außendurchmessers aufweist, von denen die elektrisch leitenden Kontakte mit jeweils einer elektrisch leitenden Anschlussleitung elektrisch leitend verbunden sind, und wobei das Anschlussstück von mehreren isolierenden Zwischenstücken (14), wobei wenigstens einige der isolierenden Zwischenstücke einander identisch sind, und von von diesen gehaltenen elektrischen Kontakten mit daran befestigten Anschlussleitungen gebildet ist, die zusammengesteckt und nach dem Zusammenstecken mittels einer thermoplastischen Spritzmasse verbunden sind, wobei das Anschlussstück im übrigen so ausgebildet ist, dass es als eigenständige Einheit mit einer übrigen Elektrodenleitung zu verbinden ist, **dadurch gekennzeichnet, dass** die einander identischen Zwischenstücke in einem jeweiligen distalen Längsabschnitt einen geringeren Außendurchmesser aufweisen, als in ihrem proximalen Längsabschnitt, wobei der Außendurchmesser im distalen Längsabschnitt so bemessen ist, dass ein jeweiliger distaler Längsabschnitt genau in eine zentrale Öffnung im proximalen Längsabschnitt eines identischen Zwischenstücks passt, so dass die Zwischenstücke auf diese Weise ineinander stecken und so miteinander verbunden sind.

2. Anschlussstück nach Anspruch 1, **dadurch gekennzeichnet, dass** die isolierenden Zwischenstücke im Innern des Anschlussstücks angeordnet sind und dass die Außenkontur des Anschlussstücks von der Spritzmasse zusammen mit den elektrisch leitenden Kontakten durch Umspritzen der Zwischenstücke mit der Spritzmasse nach dem Zusammenfügen der Zwischenstücke und der elektrischen Kontakte gebildet ist.

3. Anschlussstück nach Anspruch 1, **dadurch gekennzeichnet, dass** die isolierenden Zwischenstücke zusammen mit den elektrisch leitenden Kontakten die Außenkontur des Anschlussstücks bilden und einen Innenraum einschließen, in den die Spritzmasse nach dem Zusammenfügen der Zwischenstücke und der elektrischen Kontakte durch Spritzgießen eingebracht ist.

4. Anschlussstück nach einem der Ansprüche 1 bis 3, **dadurch gekennzeichnet, dass** die Anschlussleitungen proximal des distalen Endes des Anschlussstücks enden.

5. Anschlussstück nach einem der Ansprüche 1 bis 4, **dadurch gekennzeichnet, dass** die Spritzmasse aus einem einzigen Material besteht.

6. Anschlussstück nach Anspruch 5, **dadurch gekennzeichnet, dass** die Spritzmasse aus PEEK besteht.

7. Anschlussstück nach einem der Ansprüche 1 bis 4, **dadurch gekennzeichnet, dass** die Spritzmasse wenigstens zwei Materialen enthält.

8. Anschlussstück nach Anspruch 7, **dadurch gekennzeichnet, dass** die Spritzmasse PEEK und Polyurethan enthält.

9. Anschlussstück nach einem der Ansprüche 1 bis 8, **dadurch gekennzeichnet, dass** die einander identischen Zwischenstücke jeweils einen passgenauen Sitz für einen jeweiligen ringförmigen Kontakt aufweisen.

10. Anschlussstück nach Anspruch 9, **dadurch gekennzeichnet, dass** die einander identischen Zwischenstücke in einem jeweiligen proximalen Längsabschnitt einen Längsanschlag besitzen, der ein Aufschieben eines ringförmigen Kontaktes in proximale Richtung begrenzt.

11. Anschlussstück nach Anspruch 10, **dadurch gekennzeichnet, dass** der Längsanschlag einen Längsabstand zum proximalen Ende eines jeweiligen der identischen Zwischenstücke hat, wobei dieser Längsabstand den Längsabstand und damit auch die Isolationslänge zwischen den ringförmigen Kontakten definiert.

12. Anschlussstück gemäß einem der Ansprüche 1 bis 11, **dadurch gekennzeichnet, dass** der proximale Längsabschnitt eines jeweiligen Zwischenstücks mit seinem relativ größeren Außendurchmesser den Sitz für einen jeweiligen ringförmigen Kontakt bildet.

13. Anschlussstück nach einem der Ansprüche 1 bis 12, **dadurch gekennzeichnet, dass** die einander identischen Zwischenstücke auf ihrer Außenseite jeweils wenigstens eine in Längsrichtung verlaufende Vertiefung zur Aufnahme einer Anschlussleitung eines ringförmigen Kontaktes aufweisen.

14. Anschlussstück nach Anspruch 13, **dadurch gekennzeichnet, dass** die einander identischen Zwischenstücke einen Übergangsbereich zwischen ihrem jeweiligen proximalen Längsabschnitt und ihrem jeweiligen distalen Längsabschnitt aufweisen, in dem der Außendurchmesser abnimmt und der in Verlängerung einer jeweiligen Vertiefung einen Durchbruch aufweist, der vom Äußeren des distalen Längsabschnitts ins Innere des proximalen Längsabschnitts führt.

15. Anschlussstück nach einem der Ansprüche 1 bis 14, **dadurch gekennzeichnet, dass** das Anschlussstück ein distales Abschlussstück aufweist, welches einen proximalen Endabschnitt und eine distalen Endabschnitt besitzt, die beide annähernd den gleichen Außendurchmesser aufweisen und von denen der proximale Endabschnitt des distalen Abschlussstücks einem jeweiligen proximalen Längsabschnitt der einander identischen Zwischenstücke zwischen dem proximalen Ende des jeweiligen Zwischenstücks und den Längsanschlag desselben Zwischenstücks entspricht.

16. Anschlussstück nach Anspruch 15, **dadurch gekennzeichnet, dass** der proximale Endabschnitt des distalen Abschlussstücks auf seiner Außenseite wenigstens eine in Längsrichtung verlaufende Vertiefung zur Aufnahme einer Anschlussleitung eines ringförmigen Kontaktes aufweist.

17. Anschlussstück gemäß der Ansprüche 13 und 16, **dadurch gekennzeichnet, dass** jedes der Zwischenstücke und das distale Abschlussstück jeweils über den Umfang ihrer Längsabschnitte bzw. des distalen Endschnitts drei, über den jeweiligen Umfang gleichmäßig verteilte Vertiefungen zur Aufnahme von insgesamt drei Anschlussleitungen von ebenfalls drei ringförmigen Kontakten aufweisen, die so zueinander ausgerichtet sind, dass sie jeweils in Längsrichtung fluchten.

18. Anschlussstück nach einem der Ansprüche 1 bis 17, **dadurch gekennzeichnet, dass** das Anschlussstück ein zentrales, freies und sich über seine volle Länge erstreckendes Lumen aufweist.

19. Anschlussstück nach einem der Ansprüche 1 bis 18, **dadurch gekennzeichnet, dass** das Anschlussstück ein formschlüssiges Segment aufweist, welches in die Öffnung am proximalen Ende desjenigen der identischen Zwischenstücke passgenau eingesetzt ist, welches sich am proximalen Ende des Anschlussstücks befindet.

20. Elektrodenleitung, die an ihrem distalen Ende wenigstens eine Elektrode und wenigstens eine elektrische Zuleitung, die mit dieser Elektrode elektrisch verbunden ist, aufweist, **dadurch gekennzeichnet, dass** die elektrische Zuleitung mit einer Anschlussleitung eines ringförmigen Kontaktes über eine Crimp-, Schweiß- oder Lötverbindung elektrisch leitend verbunden ist, wobei die Elektrodenleitung ein Anschlusstück nach einem der Ansprüche 1 bis 19 aufweist.

21. Elektrodenleitung nach Anspruch 20, **dadurch gekennzeichnet, dass** die Crimp-, Schweiß- oder Lötverbindung jeweils in einer der in Längsrichtung verlaufenden Vertiefungen auf der Außenseite des distalen Endanschnitts des distalen Abschlussstücks angeordnet ist.

22. Elektrodenleitung nach Anspruch 18 und Anspruch 20, **dadurch gekennzeichnet, dass** die Elektrodenleitung eine sich zwischen ihrem distalen und ihrem proximalen Ende erstreckende, wendelförmige Leitung aufweist, die sich mit einem proximalen Leitungsendabschnitt in dem Lumen des Anschlussstücks erstreckt.

## Claims

1. A terminal piece for an electrode lead for connecting to an implantable cardiac stimulator and/or implantable defibrillator, the terminal piece having multiple annular, electrically conductive contacts (12) of identical outside diameters having insulation sections of the same outside diameters disposed between them in the longitudinal direction, each of the electrically conductive contacts being connected in an electrically conductive manner to an electrically conductive connecting line, the terminal piece being formed by multiple insulating intermediate pieces (14), of which at least some are identical to each other, and by electrical contacts held thereby having connecting lines fastened thereto, which are plugged together and are connected by way of a thermoplastic injection molding compound after being plugged together, the terminal piece otherwise being designed so that it is to be connected to a remaining electrode lead as an independent unit, **characterized in that** the intermediate pieces that are identical to each other have a smaller outside diameter in a respective distal longitudinal section than in the proximal longitudinal sections thereof, the outside diameter in the distal longitudinal section being dimensioned so that a respective distal longitudinal section fits exactly into a central opening in the proximal longitudinal section of an identical intermediate piece, so that the intermediate pieces are in this way plugged into each other and are thus connected to each other.

2. The terminal piece according to claim 1, **characterized in that** the insulating intermediate pieces are disposed in the interior of the terminal piece, and the outside contour of the terminal piece is formed by the injection molding compound together with the electrically conductive contacts by encapsulating the intermediate pieces with the injection molding compound after the intermediate pieces and the electrical contacts have been joined.

3. The terminal piece according to claim 1, **characterized in that** the insulating intermediate pieces together with the electrically conductive contacts form the outside contour of the terminal piece and enclose an inner space into which the injection molding compound is introduced by injection molding after the intermediate pieces and the electrical contacts have been joined.

4. A terminal piece according to any one of claims 1 to 3, **characterized in that** the connecting lines end proximally from the distal end of the terminal piece.

5. A terminal piece according to any one of claims 1 to 4, **characterized in that** the injection molding compound is made of a single material.

6. The terminal piece according to claim 5, **characterized in that** the injection molding compound comprises PEEK.

7. A terminal piece according to any one of claims 1 to 4, **characterized in that** the injection molding compound comprises at least two materials.

8. The terminal piece according to claim 7, **characterized in that** the injection molding compound comprises PEEK and polyurethane.

9. A terminal piece according to any one of claims 1 to 8, **characterized in that** each of the intermediate pieces that are identical to each other has a precisely fitting seat for a respective annular contact.

10. The terminal piece according to claim 9, **characterized in that** the intermediate pieces that are identical to each other have a longitudinal stop in a respective proximal longitudinal section, the stop delimiting pushing of an annular contact in the proximal direction.

11. The terminal piece according to claim 10, **characterized in that** the longitudinal stop has a longitudinal distance from the proximal end of each of the identical intermediate pieces, this longitudinal distance defining the longitudinal distance and thus also an insulation length between the annular contacts.

12. A terminal piece according to any one of claims 1 to 11, **characterized in that** the relatively larger outside diameter of the proximal longitudinal section of a respective intermediate piece forms the seat for a respective annular contact.

13. A terminal piece according to any one of claims 1 to 12, **characterized in that** the outsides of the intermediate pieces that are identical to each other each have at least one depression extending in the longitudinal direction for receiving a connecting line of an annular contact.

14. The terminal piece according to claim 13, **characterized in that** the intermediate pieces that are identical to each other each have a transition region between the respective proximal longitudinal section and the respective distal longitudinal section, the outside diameter decreasing in this transition region and this region in extension of a respective depression having an aperture, which leads from the outside of the distal longitudinal section into the inside of the proximal longitudinal section.

15. A terminal piece according to any one of claims 1 to 14, **characterized in that** the terminal piece comprises a distal end piece, which has a proximal end section and a distal end section, which both have approximately the same outside diameters and of which the proximal end section of the distal end piece corresponds to a respective proximal longitudinal section of the intermediate pieces that are identical to each other between the proximal end of the respective intermediate piece and the longitudinal stop of the same intermediate piece.

16. The terminal piece according to claim 15, **characterized in that** the outside of the proximal end section of the distal end piece has at least one depression extending in the longitudinal direction for receiving a connecting line of an annular contact.

17. A terminal piece according to claims 13 and 16, **characterized in that**, over the circumference of the longitudinal sections or of the distal end section, each of the intermediate pieces and the distal end piece have three depressions distributed evenly over the respective circumference for receiving a total of three connecting lines of likewise three annular contacts, which are oriented with respect to each other in such a way that they are each aligned in the longitudinal direction.

18. A terminal piece according to any one of claims 1 to 17, **characterized in that** the terminal piece has a central, free lumen extending over the entire length of the terminal piece.

19. A terminal piece according to any one of claims 1 to 18, **characterized in that** the terminal piece has a positively fitting segment, which is inserted with precise fit into the opening at the proximal end of that piece of the identical intermediate pieces which is located at the proximal end of the terminal piece.

20. An electrode lead, comprising at the distal end thereof at least one electrode and at least one electrical supply line, which is electrically connected to this electrode, **characterized in that** the electrical supply line is connected in an electrically conductive manner to a connecting line of an annular contact by way of a crimp, weld, or solder joint, the electrode lead comprising a terminal piece according to any one of claims 1 to 19.

21. The electrode lead according to claim 20, **characterized in that** the crimp, weld, or solder joint is located in each case in one of the depressions extending in the longitudinal direction on the outside of the distal end section of the distal end piece.

22. An electrode lead according to claim 18 and claim 20, **characterized in that** the electrode lead comprises a helical line which extends between the distal end and the proximal end of the electrode lead and the proximal lead end section of which extends into the lumen of the terminal piece.

## Revendications

1. Pièce de raccordement pour une ligne d'électrode, pour le raccordement d'un stimulateur cardiaque implantable et/ou d'un défibrillateur implantable, la pièce de raccordement comportant plusieurs contacts annulaires électriquement conducteurs (12) présentant un diamètre extérieur identique, avec des sections isolantes du même diamètre agencées entre ceux-ci dans le sens longitudinal, les contacts électriquement conducteurs étant respectivement reliés de façon électriquement conductrice à une ligne de raccordement électriquement conductrice, et la pièce de raccordement étant constituée de plusieurs pièces intermédiaires isolantes (14), certaines des pièces intermédiaires isolantes étant identiques les unes aux autres, et de contacts électriques maintenus par celles-ci, auxquels sont fixées des lignes de raccordement, lesquels sont emboîtés puis reliés suite à l'emboîtement, au moyen d'une matière thermoplastique à mouler par injection, la pièce de raccordement étant au surplus conçue pour être reliée en tant qu'unité autonome à une autre ligne d'électrode, **caractérisée en ce que** les pièces intermédiaires identiques entre elles présentent un diamètre extérieur plus petit dans une section longitudinale respectivement distale que dans leur section longitudinale proximale, le diamètre extérieur étant mesuré dans la section longitudinale distale de manière à ce qu'une section longitudinale distale respective soit exactement adaptée à une ouverture centrale dans la section longitudinale proximale d'une pièce intermédiaire identique, de sorte que les pièces intermédiaires s'emboîtent ainsi les unes dans les autres et sont ainsi reliées entre elles.

2. Pièce de raccordement selon la revendication 1, **caractérisée en ce que** les pièces intermédiaires isolantes sont agencées à l'intérieur de la pièce de raccordement et **en ce que** le contour extérieur de la pièce de raccordement est formé par la matière à mouler par injection, ensemble avec les contacts électriquement conducteurs, par enrobage par injection des pièces intermédiaires avec la matière à mouler par injection, après l'assemblage des pièces intermédiaires et des contacts électriques.

3. Pièce de raccordement selon la revendication 1, **caractérisée en ce que** les pièces intermédiaires isolantes forment le contour extérieur de la pièce de raccordement, ensemble avec les contacts électriquement conducteurs, et renferment un espace intérieur dans lequel est introduite la matière à mouler par injection, après l'assemblage des pièces intermédiaires et des contacts électriques, par moulage par injection.

4. Pièce de raccordement selon l'une des revendications 1 à 3, **caractérisée en ce que** les lignes de raccordement se terminent à proximité de l'extrémité distale de la pièce de raccordement.

5. Pièce de raccordement selon l'une des revendications 1 à 4, **caractérisée en ce que** la matière à mouler par injection est constituée d'une seule matière.

6. Pièce de raccordement selon la revendication 5, **caractérisée en ce que** la matière à mouler par injection est constituée de PEEK.

7. Pièce de raccordement selon l'une des revendications 1 à 4, **caractérisée en ce que** la matière à mouler par injection contient au moins deux matières.

8. Pièce de raccordement selon la revendication 7, **caractérisée en ce que** la matière à mouler par injection contient du PEEK et du polyuréthane.

9. Pièce de raccordement selon l'une des revendications 1 à 8, **caractérisée en ce que** les pièces intermédiaires identiques entre elles présentent respectivement une assise précise pour un contact annulaire respectif.

10. Pièce de raccordement selon la revendication 9, **caractérisée en ce que** dans une section longitudinale proximale respective, les pièces intermédiaires identiques entre elles possèdent une butée longitudinale délimitant un emboîtement du contact annulaire dans la direction proximale.

11. Pièce de raccordement selon la revendication 10, **caractérisée en ce que** la butée longitudinale présente un espacement longitudinal par rapport à l'extrémité proximale de chacune des pièces intermédiaires identiques, cet espacement longitudinal définissant l'espacement longitudinal et donc également la longueur d'isolation entre les contacts annulaires.

12. Pièce de raccordement selon l'une des revendications 1 à 11, **caractérisée en ce que** la section longitudinale proximale de chacune des pièces intermédiaires forme l'assise pour un contact annulaire respectif, avec son diamètre extérieur relativement plus grand.

13. Pièce de raccordement selon l'une des revendications 1 à 12, **caractérisée en ce que** sur leur côté extérieur, les pièces intermédiaires identiques entre elles comportent respectivement au moins un creux s'étendant dans le sens longitudinal, pour l'admission d'une ligne de raccordement d'un contact annulaire.

14. Pièce de raccordement selon la revendication 13, **caractérisée en ce que** les pièces intermédiaires identiques entre elles comportent une région de transition entre leur section longitudinale proximale respective et leur section longitudinale distale respective, dans laquelle le diamètre extérieur diminue et lequel comporte une percée dans le prolongement d'un creux respectif, laquelle mène de l'extérieur de la section longitudinale distale vers l'intérieur de la section longitudinale proximale.

15. Pièce de raccordement selon l'une des revendications 1 à 14, **caractérisée en ce que** la pièce de raccordement comporte une pièce terminale distale possédant une section d'extrémité proximale et une section d'extrémité distale, présentant toutes deux quasiment le même diamètre extérieur et parmi lesquelles la section d'extrémité proximale de la pièce terminale distale correspond à une section longitudinale proximale respective des pièces intermédiaires identiques situées entre l'extrémité proximale de la pièce intermédiaire respective et la butée longitudinale de ladite pièce intermédiaire.

16. Pièce de raccordement selon la revendication 15, **caractérisée en ce que** dans son côté extérieur, la section d'extrémité proximale de la pièce terminale distale comporte au moins un creux s'étendant dans le sens longitudinal, pour l'admission d'une ligne de raccordement d'un contact annulaire.

17. Pièce de raccordement selon les revendications 13 et 16, **caractérisée en ce que** sur le pourtour de leurs sections longitudinales ou de la section d'extrémité distale, chacune des pièces intermédiaires et la pièce terminale distale comportent respectivement trois creux répartis régulièrement sur le pourtour respectif, pour l'admission d'un total de trois lignes de raccordement de contacts annulaires également au nombre de trois, lesquels sont orientés les uns par rapport aux autres de manière à être respectivement en affleurement dans le sens longitudinal.

18. Pièce de raccordement selon l'une des revendications 1 à 17, **caractérisée en ce que** la pièce de raccordement comporte un lumen central libre s'étendant sur toute sa longueur.

19. Pièce de raccordement selon l'une des revendications 1 à 18, **caractérisée en ce que** la pièce de raccordement comporte un segment à complémentarité de forme, lequel est inséré en ajustement serré dans l'ouverture à l'extrémité proximale de celle parmi les pièces intermédiaires identiques qui se trouve à l'extrémité proximale de la pièce de raccordement.

20. Ligne d'électrode comportant à son extrémité distale au moins une électrode et au moins une ligne d'alimentation électrique reliée électriquement à cette électrode, **caractérisée en ce que** la ligne d'alimentation électrique est relié de façon électriquement conductrice à une ligne de raccordement d'un contact annulaire, à l'aide d'un raccordement par sertissage, soudage ou brasage, la ligne d'électrode comportant une pièce de raccordement selon l'une des revendications 1 à 19.

21. Ligne d'électrode selon la revendication 20, **caractérisée en ce que** le raccordement par sertissage, soudage ou brasage est réalisé respectivement dans l'un des creux s'étendant dans le sens longitudinal, du côté extérieur de la section d'extrémité distale de la pièce terminale distale.

22. Ligne d'électrode selon la revendication 18 et la revendication 20, **caractérisée en ce que** la ligne d'électrode comporte une ligne torsadée s'étendant entre son extrémité distale et son extrémité proximale, laquelle s'étend avec sa section d'extrémité de ligne proximale dans le lumen de la pièce de raccordement.
